# EUROPÄISCHE PATENTSCHRIFT

(11) **EP 1 844 714 B1**
(45) Veröffentlichungstag und Bekanntmachung des Hinweises auf die Patenterteilung: **24.10.2012**
(21) Anmeldenummer: 07007615.3
(22) Anmeldetag: 13.04.2007
(51) Int. Cl.: A61B 17/02

(54) **Medizisches Instrument zum Auseinanderspreizen von Wirbelkörpern**
Medical instrument for stretching vertebral bodies
Instrument médical destiné à maintenir une distance constante entre des vertèbres

(30) Priorität: 13.04.2006 DE 102006018248
(43) Veröffentlichungstag der Anmeldung: 17.10.2007
(73) Patentinhaber: Karl Storz GmbH & Co. KG, 78532 Tuttlingen (DE)
(72) Erfinder: Bacher, Uwe, 78532 Tuttlingen (DE); Oberländer, Martin, 78234 Engen (DE)
(74) Vertreter: Weller, Wolfgang

(56) Entgegenhaltungen:
- EP-B1- 0 767 636
- DE-U1- 29 520 928
- US-A- 5 755 732
- US-A- 6 159 215

## Beschreibung

Die Erfindung betrifft ein medizinisches Instrument zum Auseinanderspreizen von Wirbelkörpern und für Operationen im Bereich einer Wirbelsäule, mit einer einen Kanal aufweisenden Kanüle und mit distalseitig vorspringenden Elementen, die zwischen zwei benachbarten Wirbeln einbringbar sind, wobei die Elemente als spreizbare Elemente ausgebildet sind, die zu einem sich distal verjüngenden Körper zusammengelegt sind, der als ein kegelartiger Körper ausgebildet ist, und dass ein Spreizkörper vorgesehen ist, der in den kegelartigen Körper einfahrbar ist und dabei diesen spreizt, so dass die beiden benachbarten Wirbel voneinander weggeschoben und beabstandet gehalten werden.

Ein derartiger Wirbelspreizer ist aus der US-A-5 755 732 bekannt.

Dieser Wirbelspreizer weist zwei spreizbare Elemente auf, die beim Spreizen quer zur Längsachse des Schaftes voneinander wegbewegt werden. Dazu wird mittig ein Spreizkörper eingefahren, der Keilflächen aufweist, die mit Keilflächen an der Innenseite der spreizbaren Elemente zusammenwirken. Wird dieser mittige zentrale Spreizkörper nach distal verschoben, werden die beiden spreizbaren Elemente radial nach außen gedrückt. Sich radial erstreckende Rückholfedern sorgen bei einem Zurückziehen des Spreizkörpers nach proximal dafür, dass die spreizbaren Elemente sich wieder zusammenlegen.

Dieses medizinische Instrument ist als reiner Wirbelspreizer ausgebildet. Dadurch, dass der Spreizkörper mittig im Schaft bzw. der Kanüle angeordnet ist, sind nach dem Spreizen keine Manipulationen durch die Kanüle hindurch durchführbar.

Gegenstand der vorliegenden Anmeldung ist ein medizinisches Instrument, das sowohl ein Auseinanderspreizen von Wirbel körpern ermöglicht, als auch das Durchführen von Operationen, bei gespreizten Elementen, durch die Kanüle hindurch.

Eine derartige Vorrichtung in Form eines Instrumentariums für die Einsetzung eines chirurgischen Implantats ist aus der DE 697 29 140 T2 bekannt.

Derartige medizinische Instrumente werden zum Spreizen von zwei benachbarten Wirbeln bei einer Untersuchung oder einem chirurgischem Eingriff im Bereich einer Wirbelsäule, zum Beispiel beim Einführen und Einsetzen eines Implantats, verwendet.

Dazu werden zwei Elemente, die am distalen Ende der Kanüle vorstehen, zwischen zwei benachbarten Wirbeln derart eingeführt, dass die beiden benachbarten Wirbel voneinander weggeschoben werden. Durch die beiden Elemente werden die benachbarten Wirbel über die gesamte Operation im gegenseitigen Abstand gehalten. Durch den durch die Kanüle hindurchgehenden Arbeitskanal können verschiedene medizinische Instrumente und ein Implantat an den bestimmten Ort zwischen die benachbarten Wirbel gebracht werden.

Die beiden distal vorstehenden Elemente sind als streifenförmige Verlängerungen der Kanülewand ausgebildet. Distal sind diese angespitzt, um ein Eintreten der Kanüle zwischen die Wirbelkörper zu erleichtern. Die Höhe bzw. die Breite der Streifen bestimmt den Abstand auf den die Wirbel gespreizt werden können.

Aus der EP 0 767 636 B1 ist Wirbelkörperimplantat bekannt, das zwei Schenkel aufweist, die spreizbar ausgebildet sind. Wenn die Schenkel zusammengelegt sind, bilden diese einen sich nach distal verjüngenden keilartigen Körper. Eine Schraube kann von proximal eingedreht werden, wodurch die Schenkel gespreizt werden.

Es ist Aufgabe der vorliegenden Erfindung ein medizinisches Instrument der eingangs genannten Art dahingehend weiterzuentwickeln, dass sowohl ein einfaches Einführen des Instruments zwischen zwei benachbarten Wirbeln als auch ein ausreichender Operationsbereich während der Untersuchung oder des chirurgischen Eingriffs geschaffen wird.

Erfindungsgemäß wird die Aufgabe dadurch gelöst, dass ein Eindring-Element vorgesehen ist, das durch die Kanüle um den sich verjüngenden Körper hindurchführbar ist, ferner dass die spreizbaren Elemente als Lamellen ausgebildet sind, und dass bei gespreizten Lamellen und bei nach proximal aus der als Spreizkörper ausgebildeten Kanüle abgezogenen Eindring-Element der Kanal der Kanüle zum Hindurchführen von Instrumenten frei ist.

Lamellen können einfach zu dem sich distalseitig verjüngenden Körper zusammengelegt werden, indem diese radial nach innen gebogen werden.

Das Eindring-Element vereinfacht das Anzielen des Ansatzpunktes des Instrumentes und das Einführen zwischen bei benachbarte Wirbel.

Nach Spreizen der Lamellen und Abiehen der Eindring-Elementes ist der Kanal der Kanüle frei für weitere Manipulationen.

Durch den Kanal können medizinische Instrumente von proximal nach distal hindurchgeschoben werden, um eine Untersuchung oder einen Eingriff zum Abschluss zu bringen.

Diese Maßnahmen haben unter anderem den erheblichen Vorteil, dass zunächst der kegelartige Körper zwischen die Wirbel eingetrieben werden kann, und erst danach durch Einfahren des Spreizkörpers der Körper und somit die Wirbel gespreizt werden. Dies ergibt der Handhabungsperson ein besseres Gefühl für das Maß und die Stärke des Spreizvorgangs.

Der kegelförmige Körper kann auf Grund seiner Form unabhängig von seiner Drehstellung einfach angesetzt und dann zwischen die zwei eng beieinander liegenden Wirbel eingetrieben werden.

Der kegelartige Körper ist vorzugsweise aus zumindest drei spreizbaren Elementen, weiter bevorzugt aus zumindest vier und höchst bevorzugt aus zumindest sechs spreizbaren Elementen gebildet. In einer Ausführungsform wird der kegelartige Körper aus zehn Lamellen aufgebaut.

Die Zahl wird durch die Größe des Instruments bzw. die Größe der Patienten beeinflusst. Je mehr spreizbare Elemente vorhanden sind, desto geringer ist der umfängliche Freiraum zwischen den gespreizten Elementen. Durch die umfängliche Verteilung der mehreren Elemente wird das Operationsfeld nicht nur in den gegensätzlichen Richtungen der Wirbel sondern auch seitlich gespreizt, also geöffnet, so dass dem Operateur ein relativ großer Raum zum Eingriff zur Verfügung steht.

In einer weiteren Ausgestaltung der Erfindung springen die Elemente von einer Hülse vor.

Diese Maßnahme hat den Vorteil, dass die Hülse mit einer menschlichen Hand ergriffen werden kann, dabei schmiegen sich die Handfläche und die Finger geschlossen um die Hülse. Somit ist das Instrument sehr fest und sicher ergreifbar. Dadurch ist die Handhabung des medizinischen Instruments beim Einführen des sich distal verjüngenden Körpers zwischen zwei benachbarten Wirbeln ergonomisch.

In einer weiteren Ausgestaltung der Erfindung ist der sich verjüngende Körper als ein Kegelstumpf ausgebildet.

Bei dieser Ausgestaltung resultiert am distalen Ende eine Öffnung, durch die eine exakte Positionierung zwischen den Wirbeln, zum Beispiel durch einen hindurchgeführten Zieldraht, bewerkstelligt werden kann.

In einer weiteren Ausgestaltung der Erfindung ist die Kanüle als Spreizkörper ausgebildet, die in der Hülse verschiebbar aufgenommen ist.

Diese Maßnahme hat den Vorteil, dass die Kanüle zusätzlich zur Spreizfunktion eine Stütze und eine Verstärkung für die gespreizten spreizbaren Elemente darstellt. Die gespreizten Wirbel üben eine beachtliche Presskraft auf die gespreizten Elemente aus.

In einer weiteren Ausgestaltung der Erfindung ist das Eindring-Element als Stab ausgebildet.

Diese Maßnahme hat den Vorteil, dass der Stab mit einer Hand durch das Instrument und distalseitig über dieses hinausgeschoben werden kann.

In einer weiteren Ausgestaltung der Erfindung verjüngt sich das Eindring-Element in Richtung dessen distalen Endes.

Diese Maßnahme hat den Vorteil, dass die verjüngte Form des distalen Endes des Stabes das Einführen des Eindring-Elements zwischen zwei benachbarte Wirbel vereinfacht.

In einer weiteren Ausgestaltung der Erfindung ist das sich verjüngende distale Ende des Stabes als eine Spitze ausgebildet.

Diese Maßnahme hat den Vorteil, dass das als Spitze ausgebildete distale Ende des Stabes punktgenau angesetzt und eingetrieben werden kann.

In einer weiteren Ausgestaltung der Erfindung sind die Lamellen durch Schlitzen der Hülse ausgebildet.

Diese Maßnahme hat den Vorteil, dass die spreizbaren Elemente durch einen einfachen Bearbeitungsvorgang an der Hülse hergestellt werden können.

In einer weiteren Ausgestaltung der Erfindung ist jede Lamelle über ein Scharnier mit der Hülse verbunden.

Diese Maßnahme hat den Vorteil, dass die Lamellen definiert um das Scharnier verschwenkt werden, und zwar sowohl beim Nach-Innen-Schwenken zum Ausbilden des sich verjüngenden Körpers als auch beim Spreizen durch die Kanüle. Insbesondere ist das Scharnier als Filmscharnier ausgebildet.

In einer weiteren Ausgestaltung der Erfindung sind die Lamellen radial nach innen gebogen.

Diese Maßnahme hat den Vorteil, dass die Elemente sich automatisch nach dem Abziehen des Spreizkörpers wieder zum konischen Körper zusammenlegen.

In einer weiteren Ausgestaltung der Erfindung weisen die spreizbaren Elemente an ihrem distalen Ende einen äußeren Widerhaken auf.

Diese Maßnahme hat den Vorteil, dass die Widerhaken dazu dienen, definierte Krafteinleitungsstellen beim Spreizen der Elemente zu schaffen.

In einer weiteren Ausgestaltung der Erfindung ergeben die Außenseiten der Widerhaken bei aufgespreizten Elementen einen Ring, dessen Außendurchmesser größer ist als der Außendurchmesser der Hülse.

Diese Maßnahme hat den Vorteil, dass das maximale Spreizmaß größer als der Außendurchmesser der Hülse sein kann. Durch die Wahl der Form und der Höhe der Widerhaken lassen sich variable Spreizmaße erzielen. Über den entstehenden ringartigen Körper kann die Spreizkraft gleichmäßig auf die Wirbel abgeleitet werden.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand eines ausgewählten Ausführungsbeispiels im Zusammenhang mit den Zeichnungen näher beschrieben und erläutert. Es zeigen:
- Fig. 1: eine perspektivische Seitenansicht eines erfindungsgemäßen medizinischen Instruments, wobei die spreizbaren Elemente zu einem sich distal verjüngenden Körper zusammengelegt sind,
- Fig. 2: eine der Darstellung von Fig. 1 vergleichbare Darstellung, wobei ein Eindring-Element eingeschoben ist,
- Fig. 3: eine der Darstellung von Fig. 2 vergleichbare Darstellung, nachdem die spreizbaren Elemente gespreizt sind,
- Fig. 4: einen Schnitt entlang der Längsachse von Fig. 2,
- Fig. 5: einen Schnitt entlang der Längsachse von Fig. 3,
- Fig. 6: ein medizinisches Instrument vor dessen Einbringung zwischen zwei benachbarte Wirbel,
- Fig. 7: ein zwischen zwei benachbarten Wirbeln eingebrachtes medizinisches Instrument vor einem Spreizen der spreizbaren Elemente,
- Fig. 8: eine der Darstellung von Fig. 7 vergleichbare Darstellung nach dem Spreizen der spreizbaren Elemente, und
- Fig. 9: eine der Darstellung von Fig. 7 vergleichbare Darstellung, nach Abziehen des Eindring-Elements.

Ein in den Figuren dargestelltes medizinisches Instrument zum Auseinanderspreizen von Wirbelkörpern und für Operationen im Bereich einer Wirbelsäule ist in seiner Gesamtheit mit der Bezugsziffer 10 bezeichnet.

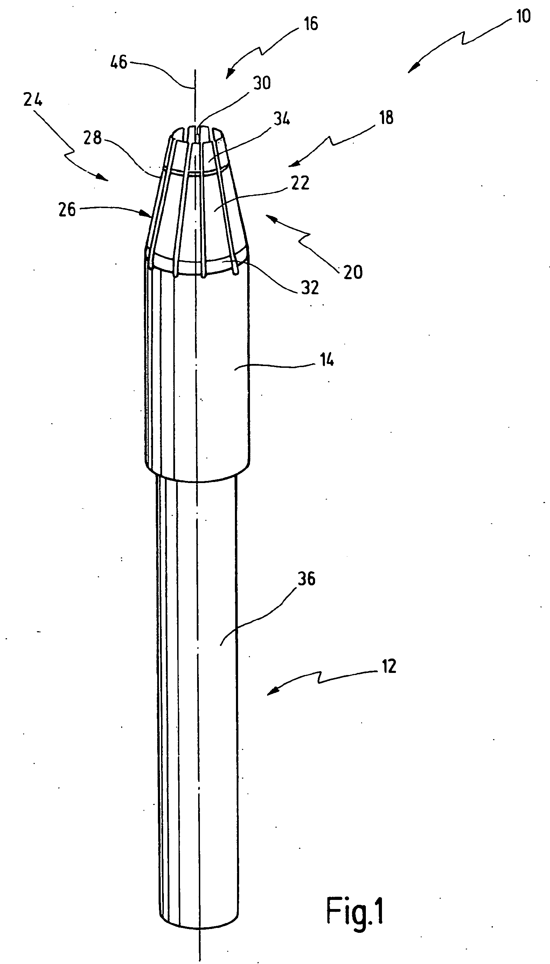

Das in Fig. 1 dargestellte medizinische Instrument 10 weist eine Kanüle 12 auf.

Die Kanüle 12 ist in einer Hülse 14 aufgenommen, die eine Länge etwa über ein Drittel der Gesamtlänge des Instruments 10 aufweist.

An einem distalen Ende 16 weist die Hülse 14 zehn umfänglich verteilte Elemente 18 auf, die als spreizbare Elemente 20 ausgebildet sind. Es können je nach Ausgestaltung auch mehr oder weniger spreizbare Elemente 20 vorhanden sein.

Die spreizbaren Elemente 20 sind als Lamellen 22 ausgebildet, die durch Schlitzen der Hülse 14 ausgebildet sind.

Die Lamellen 22 sind zu einem sich distal verjüngenden Körper 24, der als ein kegelartiger Körper 26 ausgebildet ist, zusammengelegt. Dies erfolgt beim Herstellen durch Ausfräsen.

Im dargestellten Ausführungsbeispiel ist der kegelförmige Körper 26 als ein Kegelstumpf 28 ausgebildet.

Der Kegelstumpf 28 weist an distalem Ende 16 eine Öffnung 30 auf.

Jede Lamelle 22 ist über ein Filmscharnier 32 mit der Hülse 14 verbunden. Das Filmscharnier 32 entsteht z.B. durch einen Einstich in das Material der Hülse 14.

Die Lamellen 22 werden um das Filmscharnier 32 verschwenkt.

Die Lamellen 22 bilden einen Kegelstumpf 28.

Das Material der Hülse kann ein metallisches Material oder Kunststoff sein. Die Hülse kann als Wegwerfteil ausgebildet sein.

Die Lamellen 22 weisen an ihrem distalen Ende einen äußeren Widerhaken 34 auf, die dazu dienen eine definierte Krafteinleitungsstelle beim Spreizen der Lamellen 22 zu schaffen.

Das medizinische Instrument 10 weist einen Spreizkörper 36 auf, der als Kanüle 12 ausgebildet ist, die in der Hülse 14 verschiebbar aufgenommen ist.

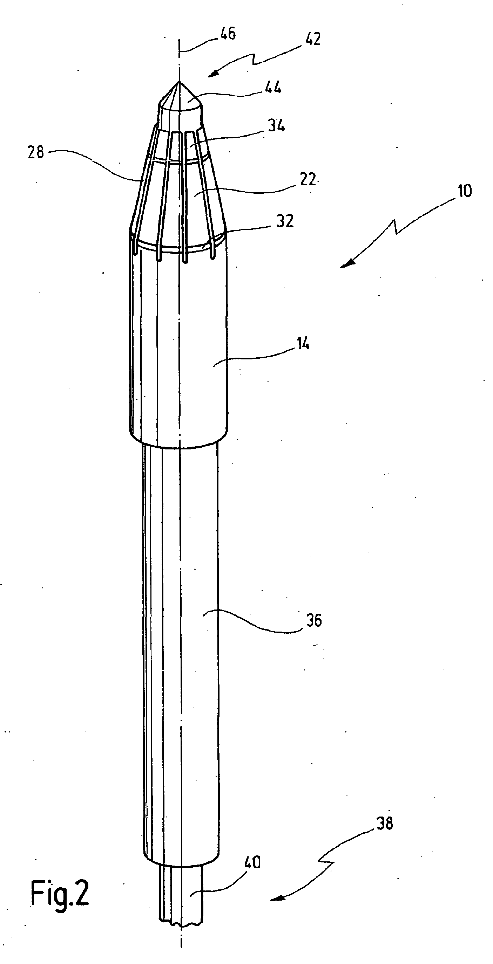

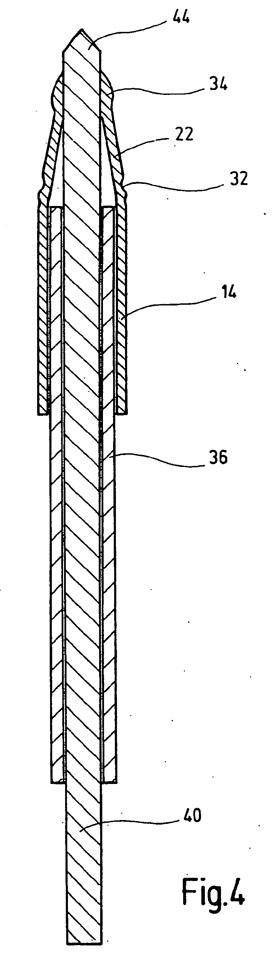

In Fig. 2 und 4 ist dargestellt, wie durch die Kanüle 12 und den Kegelstumpf 28 ein Eindring-Element 38 hindurchgeführt ist.

Das Eindring-Element 38 ist als Stab 40 ausgebildet.

Das Eindring-Element 38 verjüngt sich in Richtung dessen distalen Endes 42, und bildet eine Spitze 44 aus.

Der Kegelstumpf 28 liegt dicht an dem Stab 40 des Eindring-Elements 38 an.

Diese Konstruktion des medizinischen Instruments 10 vereinfacht das Anzielen des Ansatzpunktes des Instruments 10 und das Einführen des Kegelstumpfs 28 zwischen zwei benachbarten Wirbeln.

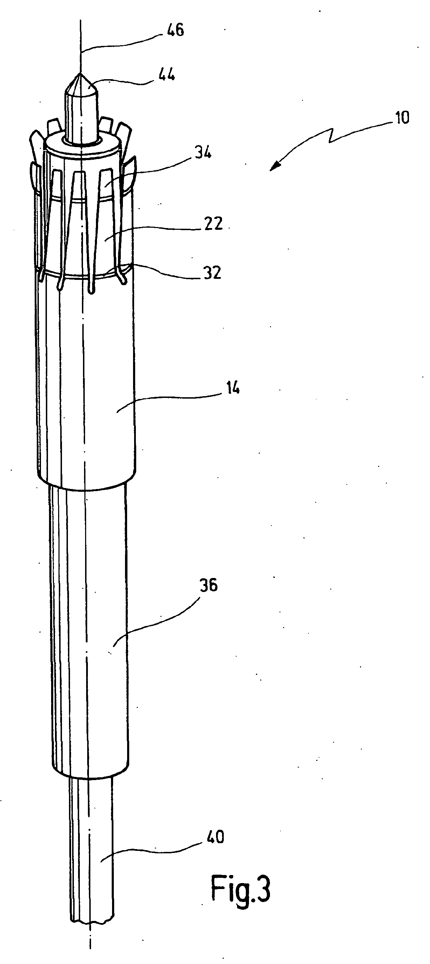

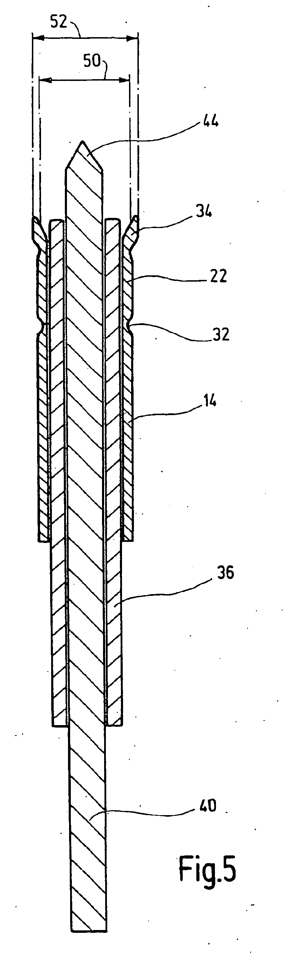

In Fig. 3 und Fig. 5 ist das medizinische Instrument 10 nach dem Spreizen der spreizbaren Elemente 20 dargestellt.

Der als Kanüle 12 ausgebildete Spreizkörper 36 wird durch eine lineare Bewegung längs einer Längsachse 46, in den sich verjüngenden Körper 24 eingefahren. Das bewirkt das Spreizen der spreizbaren Elemente 20.

Der Spreizkörper 36 stellt eine Stütze und Verstärkung für die aufgespreizten Lamellen 22 dar, auf die die Rückstellkraft der gespreizten Wirbel einwirken.

Die Darstellung von Fig. 5 verdeutlicht, dass die Außenseiten der Widerhaken 34 bei aufgespreizten Lamellen 22 einen Ring ergeben, dessen Außendurchmesser 52 größer als der Außendurchmesser der Hülse 14 ist. Die Widerhaken 34 haken sich in die Knochen der Wirbel ein und bilden definierte Krafteinleitungsstellen.

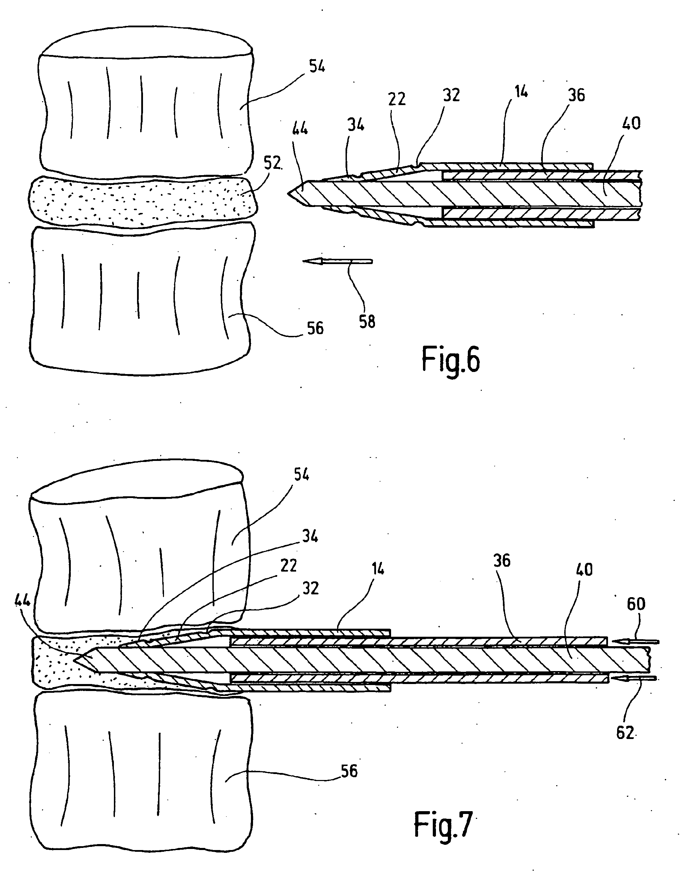

Ein Einsatz des medizinischen Instruments 10 soll im Ablauf der Figuren 6 bis 9 kurz erläutert werden.

In Fig. 6 ist eine Bandscheibe 52 dargestellt. Diese Bandscheibe 52 befindet sich zwischen zwei benachbarten Wirbeln 54 und 56 einer Wirbelsäule.

Im Fall einer Untersuchung oder eines chirurgischen Eingriffs, kommt nun das medizinische Instrument 10 folgendermaßen zum Einsatz:

Die Spitze 44 des Eindring-Elements 38 wird behutsam zwischen zwei benachbarten Wirbeln 54 und 56 angesetzt. Die Einführungsrichtung ist durch einen Pfeil 58 angezeigt.

Anschließend wird der Zusammenbau bis etwa über die gesamte Länge des Kegelstumpfs 28 zwischen die Wirbel 54 und 56 eingeführt.

Anschließend wird wie in Fig. 7 durch Pfeile 60, 62 dargestellt, der Spreizkörper 36 linear in Richtung des distalen Endes 16 bewegt.

Der Spreizkörper 36 wird dabei in den Kegelstumpf 28 eingefahren, dadurch werden die spreizbaren Elemente 20 gespreizt und die benachbarten Wirbel 54 und 56 werden dabei voneinander weggeschoben und so beabstandet gehalten. Die Spreizbewegung erfolgt gleichmäßig und definiert um die Filmscharniere 32.

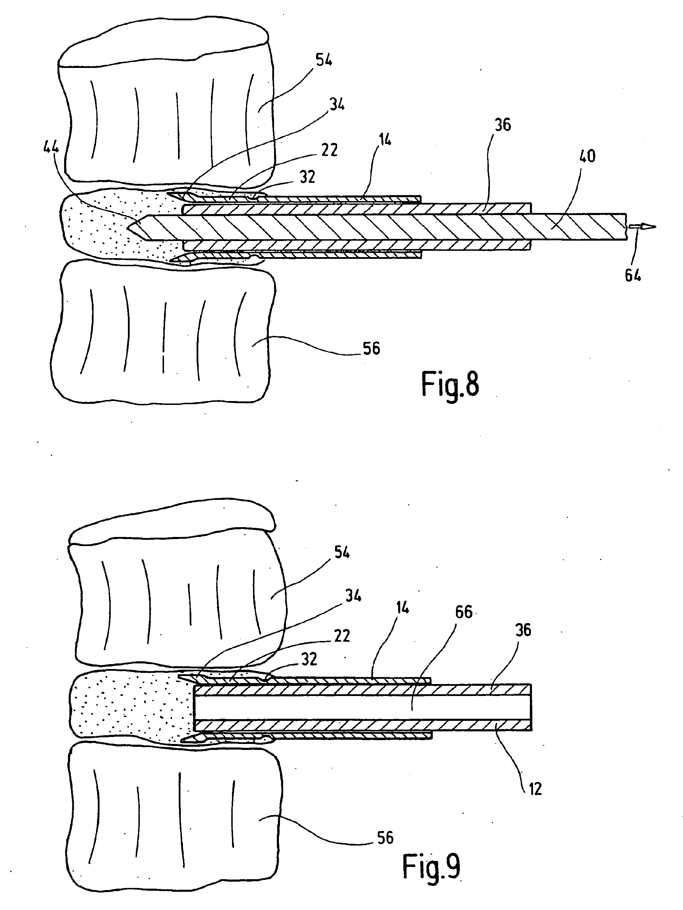

Dies ist in Fig. 8 dargestellt.

Danach wird das Eindring-Element 38 nach proximal abgezogen, wie das durch einen Pfeil 64 angezeigt ist.

Nunmehr ist ein Kanal 66 der Kanüle 12 frei für weitere Manipulationen.

Durch den Kanal 66 können nunmehr medizinische Instrumente von proximal nach distal hindurchgeschoben werden, um eine Untersuchung oder einen Eingriff zum Abschluss zu bringen.

Nach einer durchgeführten Untersuchung oder einem Eingriff wird die Kanüle 12 so weit zurückgezogen, bis die Lamellen 22 wieder radial nach innen gebogen werden und anschließend wird der Zusammenbau abgezogen.

## Patentansprüche

1. Medizinisches Instrument zum Auseinanderspreizen von Wirbelkörpern und für Operationen im Bereich einer Wirbelsäule, mit einer einen Kanal (66) aufweisenden Kanüle (12), und mit distalseitig vorspringenden Elementen (18), die zwischen zwei benachbarten Wirbeln (54, 56) einbringbar sind, wobei die Elemente (18) als spreizbare Elemente (20) ausgebildet sind, die zu einem sich distal verjüngenden Körper (24) zusammengelegt sind, der als ein kegelartiger Körper (26) ausgebildet ist und wobei ein Spreizkörper (36) vorgesehen ist, der in den kegelartigen Körper (24) einführbar ist und dabei diesen spreizt, so dass die beiden benachbarten Wirbel (54, 56) voneinander weggeschoben und beabstandet werden, **dadurch gekennzeichnet, dass** ein Eindring-Element (38) vorgesehen ist, das durch die Kanüle (12) und den sich verjüngenden Körper (24) hindurchführbar ist, dass die spreizbaren Elemente (20) als Lamellen (22) ausgebildet sind, und dass bei gespreizten Lamellen (22) und bei nach proximal aus der als Spreizkörper (36) ausgebildeten Kanüle (12) abgezogenem Eindring-Element (38) der Kanal (66) der Kanüle (12) zum Hindurchführen von Instrumenten frei ist.

2. Medizinisches Instrument nach Anspruch 1, **dadurch gekennzeichnet, dass** die spreizbaren Lamellen (22) von einer Hülse (14) vorspringen.

3. Medizinisches Instrument nach Anspruch 1 oder 2, **dadurch gekennzeichnet, dass** der sich verjüngende Körper (24) als ein Kegelstumpf (28) ausgebildet ist.

4. Medizinisches Instrument nach Anspruch 2 oder 3, **dadurch gekennzeichnet, dass** die Kanüle (12) als Spreizkörper (36) ausgebildet ist, die in der Hülse (14) verschiebbar aufgenommen ist.

5. Medizinisches Instrument nach Anspruch 1 **dadurch gekennzeichnet, dass** das Eindring-Element (38) als Stab (40) ausgebildet ist.

6. Medizinisches Instrument nach Anspruch 1 oder 5, **dadurch gekennzeichnet, dass** sich das Eindring-Element (38) in Richtung dessen distalen Endes (16) verjüngt.

7. Medizinisches Instrument nach Anspruch 6, **dadurch gekennzeichnet, dass** das sich verjüngende distale Ende (42) des Stabes (40) als Spitze (44) ausgebildet ist.

8. Medizinisches Instrument nach Anspruch 1, **dadurch gekennzeichnet, dass** die Lamellen (22) durch Schlitzen einer Hülse (14) ausgebildet sind.

9. Medizinisches Instrument nach Anspruch 1 oder 8, **dadurch gekennzeichnet, dass** jede Lamelle (22) über ein Scharnier (32) mit der Hülse (14) verbunden ist.

10. Medizinisches Instrument nach einem der Ansprüche 1 bis 9, **dadurch gekennzeichnet, dass** die Lamellen (22) radial nach innen gebogen sind.

11. Medizinisches Instrument nach einem der Ansprüche 1 bis 10, **dadurch gekennzeichnet, dass** die spreizbaren Lamellen (22) an ihrem distalen Ende einen äußeren Widerhaken (34) aufweisen.

12. Medizinisches Instrument nach Anspruch 11, **dadurch gekennzeichnet, dass** die Außenseiten der Widerhaken (34) bei aufgespreizten Lamellen (22) einen Ring ergeben, dessen Außendurchmesser (48) größer ist als der Außendurchmesser (50) der Hülse.

## Claims

1. Medical instrument for spreading vertebral bodies apart and for operations in the area of a spinal column, with a cannula (12) having a channel (66), and with elements (18) that protrude from the distal end and which can be inserted between two adjacent vertebrae (54, 56), wherein the elements (18) are designed as spreadable elements (20) that are folded together to form a distally tapering body (24) which is designed as a conical body (16), and wherein a spreader (36) is provided which can be driven into the tapering body (24) and thereby spreads the latter, such that the two adjacent vertebrae (54, 56) are pushed away from one another and kept spaced apart, **characterized in that** a penetrating element (38) is provided that can be guided through the cannula (12) and through the tapering body (24), and **in that** the spreadable elements (20) are designed as blades (22), and **in that** with spreaded blades and with the penetrating element (38) withdrawn in a proximal direction from the cannula (12) which is designed as a spreader (36), the channel (16) in the cannula (12) is free for passing instruments therethrough.

2. Medical instrument of claim 1, **characterized in that** the spreadable blades (22) protrude from a sleeve (14).

3. Medical instrument of claims 1 or 2, **characterized in that** the tapering body (24) is designed as a truncated cone (28).

4. Medical instrument of claims 1 or 3, **characterized in that** the cannula (12) is designed as a spreader (36) which is received displaceably in the sleeve (14).

5. Medical instrument of claim 1, **characterized in that** the penetrating element (38) is designed as a rod (40).

6. Medical instrument of claim 1 or 5, **characterized in that** the penetrating element (38) tapers in the direction of its distal end (16).

7. Medical instrument of claim 6, **characterized in that** the tapering distal end (42) of the rod (40) is designed as a point (44).

8. Medical instrument of claim 1, **characterized in that** the blades (22) are formed by slitting a sleeve (14).

9. Medical instrument of any one of claims 1 through 8, **characterized in that** each blade (22) is connected to the sleeve (14) via a hinge (32).

10. Medical instrument of any one of claims 1 through 9, **characterized in that** the blades (22) are bent radially inward.

11. Medical instrument of any one of claims 1 through 10, **characterized in that** the spreadable blades have an outer barb (34) at their distal end.

12. Medical instrument of claim 11, **characterized in that** when the blades (22) are spread open, the outer faces of the barbs (34) form a ring whose external diameter (48) is greater than the external diameter of the sleeve (50).

## Revendications

1. Instrument médical destiné à l'écartement de corps vertébraux et à des opérations dans la zone de la colonne vertébrale, comportant une canule (12) présentant un canal (66) et comportant des éléments (18) faisant saillie côté distal, qui peuvent être introduits entre deux vertèbres contiguës (54, 56), où les éléments (18) sont conçus comme des éléments écartables (20) qui sont assemblés d'une part en un corps (24) se rétrécissant vers l'extrémité distale qui est exécuté en tant que corps conique (26), et où est prévu un corps d'écartement (36) qui peut être introduit dans le corps conique (24) et ce faisant l'écarte, si bien que les deux vertèbres (54, 56) contiguës sont poussées et écartées l'une de l'autre, **caractérisé en ce qu'**est prévu un élément de pénétration (38) qui peut être guidé par la canule (12) et le corps se rétrécissant (24), **en ce que** les éléments écartables (20) sont exécutés sous forme de lamelles (22) et **en ce que**, lorsque les lamelles (22) sont écartées et lorsque l'élément de pénétration (38) est retiré de la canule (12) exécutée en tant que corps d'écartement (36) vers la partie proximale, le canal (66) de la canule (12) est libéré pour y guider des instruments.

2. Instrument médical selon la revendication 1, **caractérisé en ce que** les lamelles (22) écartables font saillie d'une douille (14).

3. Instrument médical selon la revendication 1 ou 2, **caractérisé en ce que** le corps (24) se rétrécissant est exécuté sous la forme d'un cône tronqué.

4. Instrument médical selon la revendication 2 ou 3, **caractérisé en ce que** la canule (12) est exécutée en tant que corps d'écartement (36) qui est logé de façon à pouvoir être déplacé dans la douille (14).

5. Instrument médical selon la revendication 1, **caractérisé en ce que** l'élément de pénétration (38) est exécuté sous la forme d'une baguette (40).

6. Instrument médical selon la revendication 1 ou 5, **caractérisé en ce que** l'élément de pénétration (38) se rétrécit dans la direction de son extrémité distale (16).

7. Instrument médical selon la revendication 6, **caractérisé en ce que** l'extrémité distale qui se rétrécit (42) de la baguette (40) est exécutée sous la forme d'une pointe (44).

8. Instrument médical selon la revendication 1, **caractérisé en ce que** les lamelles (22) sont exécutées sous la forme de fentes d'une douille (14).

9. Instrument médical selon la revendication 1 ou 8, **caractérisé en ce que** chaque lamelle (22) est reliée à la douille (14) par une charnière (32).

10. Instrument médical selon l'une des revendications 1 à 9, **caractérisé en ce que** les lamelles (22) sont recourbées radialement vers l'intérieur.

11. Instrument médical selon l'une des revendications 1 à 10, **caractérisé en ce que** les lamelles écartables (22) présentent une barbe externe (34) au niveau de leur extrémité distale.

12. Instrument médical selon la revendication 11, **caractérisé en ce que** les côtés externes des barbes (34), lorsque les lamelles (22) sont écartées, forment un anneau dont le diamètre externe (48) est plus grand que le diamètre externe (50) de la douille.
